# NEW EUROPEAN PATENT SPECIFICATION

(11) **EP 1 296 615 B2**
(45) Date of publication and mention of the opposition decision: **13.06.2012**
(45) Mention of the grant of the patent: 23.08.2006
(21) Application number: 01939926.0
(22) Date of filing: 05.06.2001
(51) Int. Cl.: A61F 2/06

(54) **INTRAVASCULAR STENT WITH improved COATING RETAINING CAPACITY**
INTRAVASKULÄRER STENT MIT verbessertem Haltevermögen einer Beschichtung
STENT INTRAVASCULAIRE DOTE D'UNE CAPACITE DE RETENTION DE FILM AMELIOREE

(30) Priority: 05.06.2000 US 209255 P
(43) Date of publication of application: 02.04.2003
(60) Divisional application: 06015527.2
(73) Proprietor: Boston Scientific Limited, St. Michael, Barbados, West Indies (BB)
(72) Inventor: JANG, G., David, Redlands, CA 92374 (US)
(74) Representative: Vossius & Partner
(86) International application number: PCT/US2001/018419
(87) International publication number: WO 2001/093781

(56) References cited:
- EP-A- 0 950 386
- EP-A2- 0 875 217
- WO-A-98/23228
- WO-A-98/36784
- WO-A-99/23977
- WO-A1-97/33534
- WO-A1-99/49928
- WO-A1-02/098326
- WO-A2-01/66036
- WO-A2-01/93781
- US-A- 5 972 027
- US 60209255 P

## Description

This invention relates generally to intravascular stents, and more particularly to intravascular stents that include a plurality of cavities formed on a surface of the stent and are coated with a restenosis inhibiting agent.

### Description of the Related Art:

By 1999, the percutaneous balloon angioplasty and stent implant procedures have become the dominant non-surgical revascularization method of the atherosclerotic stenosis, or obstruction, of the vascular lumen, and particularly in the coronary vascular system in the heart. With balloon angioplasty alone, without use of stent, the restenosis rate after angioplasty has been as high as 25-45% in the first time clinical cases. With use of stents after balloon angioplasty, the restenosis has been reduced significantly. Even so, the restenosis rate after stent implant is reported as 10-25% range depending on the condition of the vessel stented or what specific stent was used, requiring a need for further restenosis reducing measures after intravascular stenting.

To further reduce the restenosis rate after stent implant, numerous means has been tried, including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient and costly. Mainly because it is radioactive device and radiation therapy specialist from another department has to be involved with the interventional cardiologist in the cardiac catheterization laboratory. The laser and atherectomy devices proved to be marginally useful in this purpose with added costs.

The local drug therapy appears be a very promising method for the future, as better pharmaceutical, chemical or biogenetic agents are developed and became available. Some research data, both from animal tests and human clinical studies, indicate that there are evidences of suppressing restenosis after stent implant when certain growth blocking pharmaceutical agents available today are used to coat the stent. In another instances, it has been speculated that certain surface modifying materials coated on the surface of the stent may be beneficial by it alone or in combination with growth suppressing agent, in reducing restenosis rate. In either instance, the drug or substance should be locally attached or coated on the stent and in sufficient amounts. However, attaching or coating a sufficient amount of a substance or drug on the coronary stent is not so easy a proposition.

Coating a drug or an agent on the surface of the stent has a demanding problem of enough volume of such substance coated on the small surface areas of stent struts, without increasing the physical width or thickness of stent struts. This demand directly conflicts with the metal fraction issue of the stent. If the width (and lesser degree the thickness) of stent struts is increased in order to widen drug coating surface areas, it would have an elevated deleterious foreign body effect of the increased metal fraction of the stent, which would promote restenosis.

Designing an ideal stent, particularly the coronary stent, is a very demanding balance of a numerous conflicting factors. An ideal stent requires an ideal balance of numerous different stent features built into the stent. One of the many requirements of a coronary, or any vascular stent, is to keep the metal fraction of the stent low. This means that drug coating is a very demanding task. Enough amounts of a drug or agent should be coated on the miniscule surface areas of the stent struts, in order to have the desired drug results of reducing restenosis. An average stent, particularly a coronary stent, will have problem of providing desired amount of drug-retaining capacity on the surface areas of the stent struts.

The main invention of this application is not an invention of the stent itself. The present invention is the particular measures designed to increase drug coating or attachment capacity of a stent by adding exposed surface areas or reservoir capacity of the stent, without increasing the width or thickness of the stent struts or without increasing the metal fraction of the stent. These special measures of present invention will enhance the coating substances to a stent. Further, the present invention will enhance the reservoir capacity of the stent for different forms of restenosis reducing proteins, chemicals or drugs, and will prolong the releasing time duration of the substances. U.S. patent No. 6,190,404 discloses an intravascular stent with an outer surface, an inner surface and grooves formed in the inner surface of the stent. The grooves are positioned and provided to increase the rate of migration of endothelial cells upon the inner surface of the stent.

WO 98/36784 figures 7 and 8 disclose a stent with arms having holes wherein bioactive material is contained.

EP 0 950 386 A2 discloses a strut having tapped blind holes disposed on expansion struts for delivering rapamycin.

WO 98/23228 discloses a directional drug delivery stent containing a biologically active agent. The active agent may be diffused to the walls of a body lumen, such as a blood vessel, through directional delivery openings arranged on an outer surface of the stent.

There is a need for a stent with a geometry that provides for an increased amount of a coating substance. There is a further need for a stent that includes reservoirs for retaining coatings.

The technical problem of the invention is to provide a stent having improved reservoirs for coatings. These and other objects of the present invention are achieved in an expandable stent according to claim 1. A tubular structure includes an outer surface positionable adjacent to a vessel wall and an inner surface facing a lumen of a body passageway. The tubular structure further includes a plurality of expansion struts, connector struts and cells. The tubular structure has a first diameter which permits intraluminal delivery of the tubular structure into the body passageway, and a second expanded and deformed diameter which is achieved upon the application of a radially, outwardly extending force. A plurality of cavities are formed in the outer surface of the stent and extending through the inner surface.

In another embodiment of the present invention, a method of manufacturing an intravascular stent is provided. The intravascular stent has an inner surface and an outer surface. A plurality of cavities are formed on the outer surface and extends to the inner surface of the stent. A coating substance that inhibits restenosis is formed on at least a portion of the outer surface and on at least a portion of the plurality of cavities.
Figure 1 is a flat cut-open, two-dimensional, schematic view of one embodiment of a stent of the present invention that includes cavities formed in the body of the stent.
Figure 2 is a close-up view of the stent from Figure 1 with cavities that extend from the outer surface of the stent into an interior of the stent.
Figure 3 is a cross-section, side view of a stent of the present invention with cavities that extend from the outer surface through the inner surface.
Figure 3(b) does not fall under the invention.
Figure 3(c) is a cross-sectional, magnified view of another embodiment of the present invention illustrating a stent that includes cavities that extent at a slant angle from the outer surface through the inner surface.
Figure 3(d) does not fall under the invention.
Figure 4 is a flat cut-open, two-dimensional, schematic view of a stent seen from the outer surface of the stent cavities distributed in an even pattern
Figure 5 is a close-up, magnified, view of the expansion and connector struts from Figure 4 with micro-slits and micro-holes that extend from the outer surface of the stent struts.

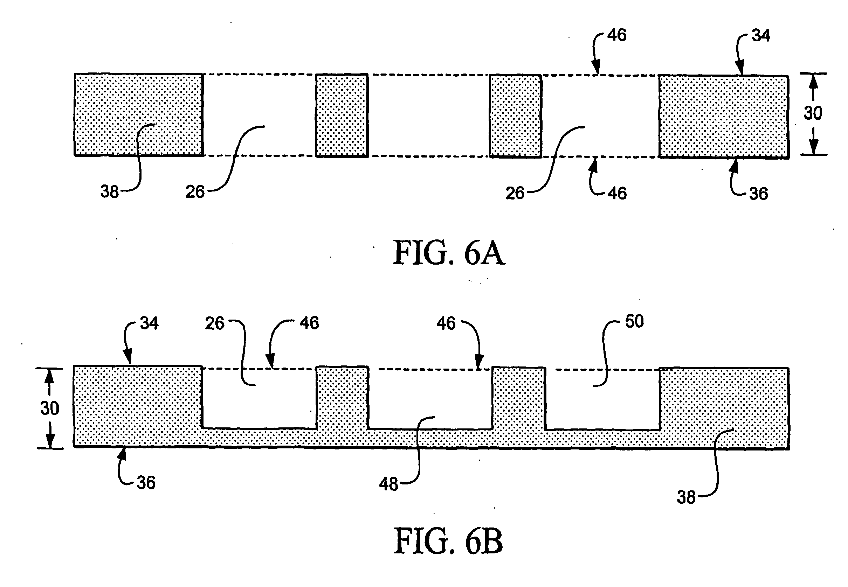

Figure 6(a) is a cross-sectional, magnified, side view of a stent strut of the present invention illustrating micro slits that extend through both the outer and inner surfaces and the entire thickness of the stent strut.
Figure 6(b) does not fall under the invention.

### DETAILED DESCRIPTION OF THE PREFERRED EMBODIMENTS

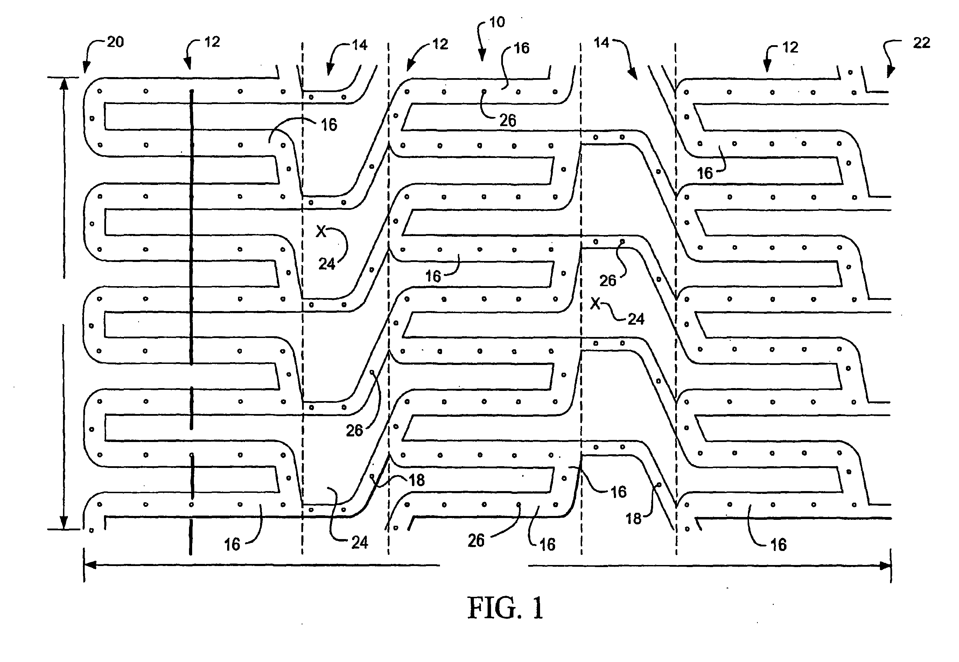

Referring now to Figure 1, one embodiment of an expandable stent 10 of the present invention is illustrated. A tubular structure includes an outer surface positionable adjacent to a vessel wall and an inner surface facing a lumen of a body passageway. The tubular structure further includes a plurality of expansion struts, connector struts and cells. The tubular structure has a first diameter which permits intraluminal delivery of the tubular structure into the body passageway, and a second expanded and deformed diameter which is achieved upon the application of a radially, outwardly extending force.

A plurality of cavities are formed in the outer surface of the stent. The cavities can be micro-holes or micro-slits and extend from the outer surface to an interior of the struts, or extend from the outer surface all the way through the inner surface. An example of a stent design useful with the present invention is disclosed in US Patent 5,954,743. In Figure 1, a two-dimensional view of the stent 10 is illustrated and is seen from the outer surface of the cut-open, two-dimensional view.

Stent 10 includes expansion columns 12 and connector columns 14 in a continuous and alternating pattern to form a longitudinal dimension and a vertical dimension. The vertical and longitudinal dimensions determine the circumference and the length respectively of stent 10. Expansion columns 12 have expansion struts 16 in a vertical zigzag or corrugated pattern. One expansion column 12 is linked to an adjacent expansion column 12 by connector column 14 between two adjacent expansion 12 columns. Connector columns 14 have connector struts 18 that serve as linking arms between expansion struts 16 in two adjacent expansion columns 12. Stent 10 has a proximal end 20 and a truncated end 22 in the middle of stent 10.

In one embodiment, stent 10 is a tubular structure that includes patterned expansion struts 16 and connectors struts 18 continuously linked circumferentially and longitudinally with a predetermined length. The total surface areas of struts 16 and 18 are limited to a certain percent of the total cylindrical surface area of tubular stent 10, particularly when stent 10 is expanded in a vessel, with enlarged (by stent expansion) stent cells 24 that make up the remainder of the total stent surface area.

The amount of a coating substance applied to and retained by stent 10 is determined by the total surface area of stent struts 16 and 18. Coating substance is preferably a restenosis inhibiting agent that is a drug, polymer and bio-engineered material and combinations thereof. It will be appreciated that other types of coating substances, well known to those skilled in the art, can be applied to stent 10 of the present invention. Because the total stent strut surface areas are limited in size, the amount of coating substance applied to stent 10 is limited to a small volume. When stent 10 is expanded in a vessel the relative surface area of struts 16 and 18 decreases in relation to the areas of stent cells 24. The total cylindrical surface area of stent 10 when it is implanted and expanded inside of a vessel is equal to the sum of the strut surface areas, which do not change, and stent cells 24 areas. The size of stent cells 24 areas changes when stent 10 is expanded. The present invention increases the amount of the coating substance capacity of stent 10 without increase the metal fraction of stent 10.

In various embodiments, the present invention increases the coating substance retaining capacity of stent 10 by forming cavities that can be micro holes 26 which are made, punched, drilled or burned into the expansion and connector struts 16. In Figure 1, micro holes 26 have openings 28 on outer surface of struts 16 and 18. Micro holes 26 are made and arranged in such a way so that they can be evenly distributed in struts 16 and 18. In this embodiment, micro holes 26 are evenly distributed through out the entire body of stent 10. The number of micro holes 26 illustrated in Figure-1 is only by example.

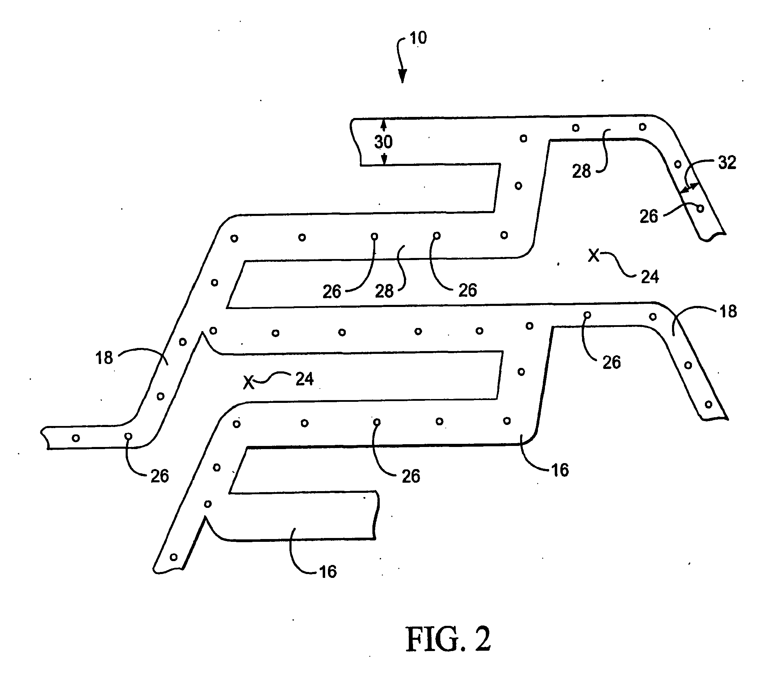

The number of micro holes 26 created in stent 10 can vary by increasing or decreasing the number according to the necessity and requirements when such stents are fabricated for clinical use. Additionally, the pattern of creating micro holes 26 in stent struts 16 and 18 can be varied according to the clinical and protocol needs. Although micro holes 26 in Figure 1 are made in straight lines it will be appreciated that micro holes 26 can be made in any varied pattern or shape. Micro-holes 26 can be made to form any suitable shape or pattern as necessary, if they meet the structural or engineering requirements of stent 10. Micro holes 26 can be made in single line or in multiple lines in struts 16 and 18 and arranged in any pattern.
In the embodiment illustrated in Figure 2, width 30 of expansion strut 16 is shown as being larger than width 32 of connector strut 18. It will be appreciated that the relative widths can change and that width 32 can be greater than width 30.

The size of micro holes 26 can be based on the physical dimensions of struts 16 and 18. Micro holes 26 cannot have diameters or size as large as the width of struts 16 and 18. Micro holes 26 can have substantially smaller widths or diameters than widths 30 and 32 in order to maintain the structural integrity and radial strength of stent 10. In one embodiment, micro holes 26 have an effective size or diameter to provide an optimal retaining capacity of substances or drugs that are coated or deposited on stent 10. Similarly, the distance between micro holes 26 is selected to maintain the integrity of stent 10 while providing an optimal number of micro holes 26 to provide a sufficient coating substance retaining capacity. Micro holes 26 in struts 18 are made smaller than micro holes 26 in expansion struts 16 or visa versa.

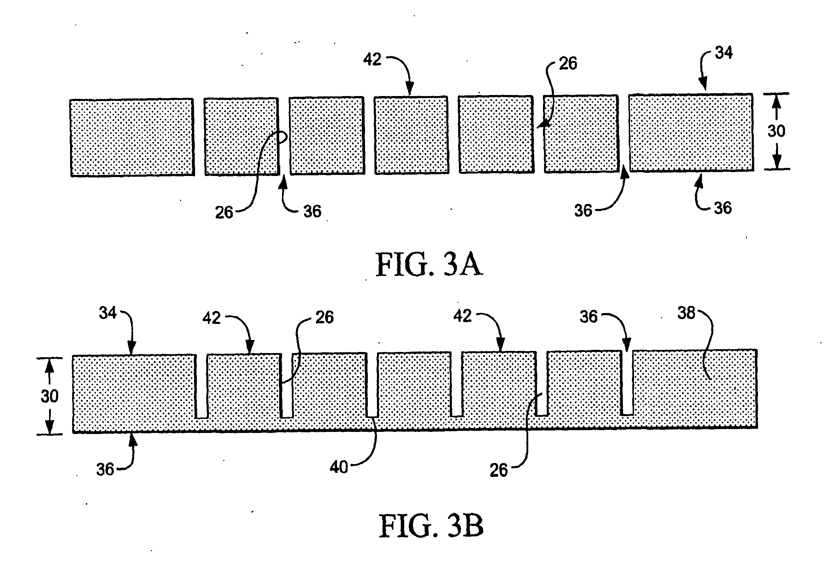

Micro holes 26 and opening 28 can have more than one shape including but not limited to circular, square, oval, oblong, irregular, polygonal or a combination thereof, depending on the method used to create micro holes 26. The tools to create micro holes 26 can be mechanical, photochemical, laser, EDM and the like.. The shape or configuration of micro holes 26 and openings 28 in stent struts 16 and 18 can be influenced by the size or diameter of the micro hole 26 made, as well as by other manufacturing factors such as a laser beam size, photochemical resolution or EDM cathode and the like. In the embodiment of Figure 3(a), micro holes 26 penetrate the entire widths 30 and 32 of struts 16 and 18 at a perpendicular angle with opening 28 on both outer surface 34 and inner surface 36. Shaded areas 38 show the cross-sectional cut surface of struts 16 and 18. Micro holes 26 are created in a regular interval with the uninterrupted segment 28 between micro holes 26. Micro holes 26 communicate freely between outer surface 34 and inner surface 36. As can be seen, micro holes 26 increase the contact surface areas of stent struts 16 or 18 for the purpose of increasing the capacity of retaining the intended coating substance added to stent 10. The bore space of micro holes 26 also serve as micro reservoir chambers for the substance to be added, attached or coated to stent 10. When stent 10 is electropolished, the shape or dimension of micro holes 26 can be slightly changed.

Referring now to Figure 3(c), micro holes 26 are shown with their axes at a slant angle relative to stent struts 16 and 18. In this embodiment, micro holes 26 extend from outer surface 34 to inner surface 36. Because micro holes 26 have a slant angle through in this embodiment, the length and reservoir capacity of the micro holes 26 is increased compared to the capacity of the Figure 3(a) micro holes 26.

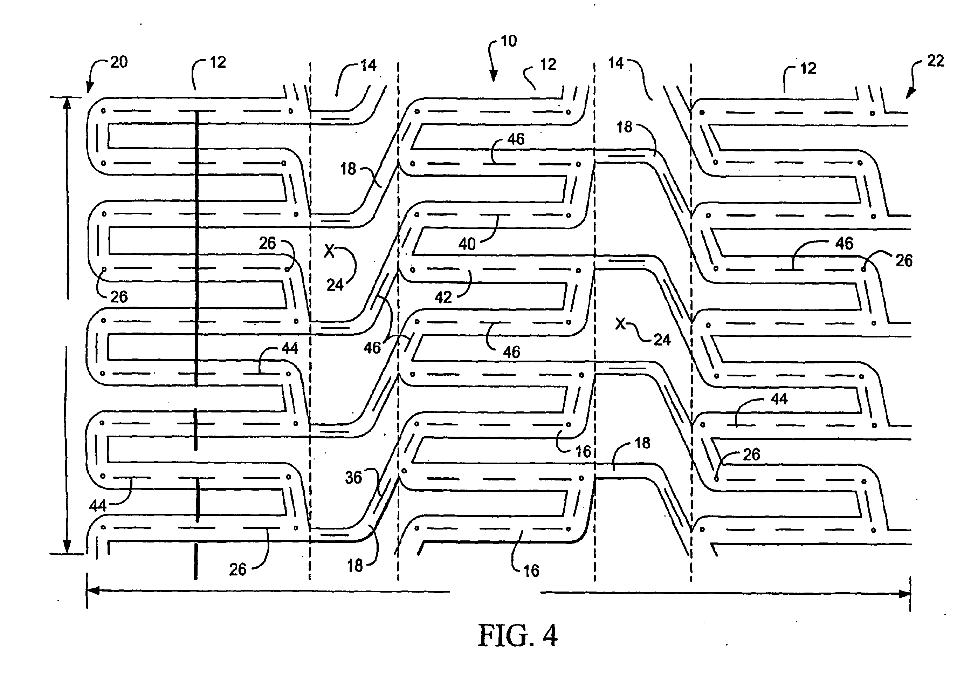

In contrast to Figure 1, the cavities formed in Figure 4 are micro slits, groves, and the like, collectively denoted as 40, which have widths 44 that are larger than openings 36. Compared to micro holes 26 of Figure 1, the micro slits 40 shown in Figure 4 provide a larger reservoir capacity for the coating substance.
Micro slits 40 can be evenly or unevenly distributed in struts 16 and 18.. The number of micro slits 40 illustrated in Figure-4 is only by way of example. Increasing or decreasing the number of micro slits 40 created in stent 10 may vary according to the clinical and pharmcodynamic necessity and requirements. Additionally, the pattern of creating micro slits 40 in struts 16 and 18 can vary according to the clinical and engineering needs. Although micro slits 40 illustrated in Figure-4 are in straight lines, micro slits 40 can be made in curvilinear, square-angles, slant angled and the like with or without radius of curvature. Micro slits 40 can be made in any suitable pattern or shape as required, if they meet the structural or engineering requirements of stent 10. Micro slits 40 can be made in single line or in multiple lines in struts 16 and 18 and arranged in any other pattern. The Figure-4 embodiment illustrates that micro holes 26 can also be included in the same stent 10.

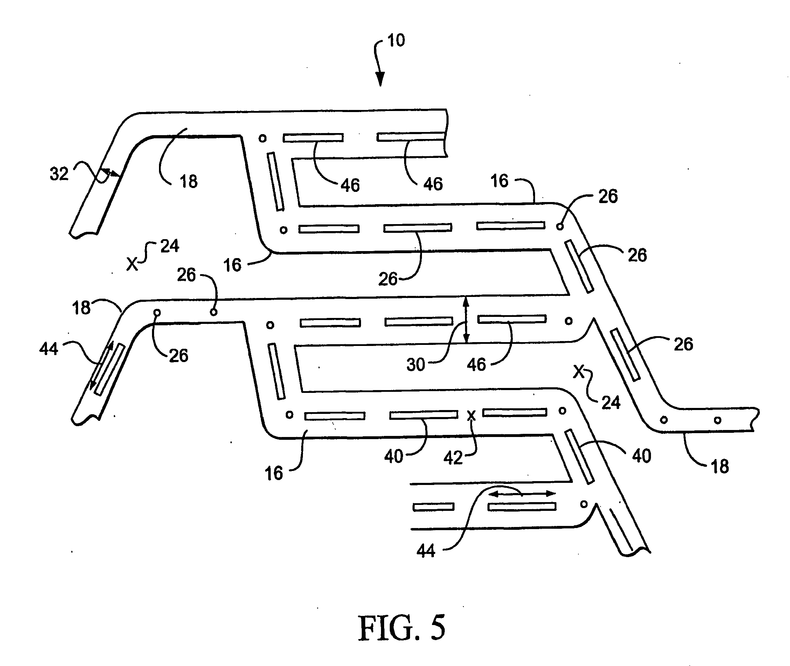

The magnified view of stent 10 illustrated in Figure 5 shows struts 16 and 18 with micro slits 40 and openings 46 on outer surface 34 of stent 10. Width 34 of expansion strut 16 is larger than width 32 of strut 18 in this embodiment. However, widths 34 and 32 can be the same in size or even reversed.

Width 44 of micro slit 40 is determined by the physical dimensions and limits of struts 16 and 18. Width 44 can not be made as large as widths 34 and 32. Width 44 is substantially smaller than width 34 in order to maintain the structural integrity and radial strength of stent 10. The length of micro slit 40 can be made as long as stent struts 16 and 18. The length of micro slit 40 can be shorter or longer than the width of struts 16 and 18. Similarly, the uninterrupted distance 42 between micro slits 40 is selected so that the structural integrity of stent 10 is not compromised. Within the allowable limits, micro slits 40 can be made in different sizes or dimensions in same or differing patterns. Micro slits 40 in struts 18 are made smaller than or greater than micro-slits 40 formed in struts 16.

The shape of micro slits 40 and opening 46 can be different than that illustrated in Figure 5. The geometry of micro slits 40 can be straight linear, curvilinear, angled, squared or any other shape, depending on the design of stent 10 and the method used to make micro slits 40 during the manufacturing process. The tools to create the micro slits 40 can be the same as those used for micro-holes 26. Different shapes, sizes and positions of micro slits 40 can be included in an individual stent 10.

The description of a preferred embodiment of the invention has been presented for purposes of illustration and description. It is not intended to be exhaustive or to limit the invention.

## Claims

1. An expandable stent (10), comprising:
• a tubular structure including an outer surface positionable adjacent to a vessel wall, an inner surface facing a lumen of a body passageway, a plurality of expansion struts (16), connector struts (18) and cells (24), the expansion struts (16) forming expansion columns (12), the tubular structure having a first diameter which permits intraluminal delivery of the tubular structure intro the body passageway having a lumen, and a second diameter in the expanded shape of the stent upon the application from the interior of the tubular structure of a radially, outwardly extending force; and
• a plurality of cavities (26, 40) formed in the expansion struts (16) and the connector struts (18) of the stent providing as reservoirs of a coating substance, wherein the cavities extend from the outer surface of the stent through the inner surface of the stent, wherein the cavities formed in the connector struts (18) are of a first size and the cavities in the expansion struts (16) are of a second size different from the first.

2. The stent of claim 1, wherein the tubular structure is balloon expandable.

3. The stent of claim 1, wherein the tubular structure is self-expandable.

4. The stent of claim 1, wherein at least a portion of the tubular structure is made of a shape memory alloy.

5. The stent of claim 1, wherein the plurality of cavities (26, 40) are substantially evenly positioned on the tubular structure.

6. The stent of claim 1 wherein the plurality of cavities increase a flexibility of the stent (10) without substantially reducing a radial strength of the stent in a deployed state.

7. The stent of claim 1, wherein the plurality of cavities (26, 40) are micro-holes that extend from the outer surface.

8. The stent of claim 7, wherein the micro-holes have a cross section that is smaller than a cross section of a strut.

9. The stent of claim 1, further comprising micro-holes extending from the outer surface to an interior of the tubular structure without extending through the inner surface.

10. The stent of claim 7, wherein at least a portion of the micro-holes have geometries that are configured to provide the reservoir .

11. The stent of claim 7, wherein at least a portion of the micro-holes have a diameter of at least 17.78 µm (0.0007 inch).

12. The stent of claim 7, wherein at least a portion of the micro-holes extend perpendicular from the outer surface to an interior of the tubular structure.

13. The stent of claim 7, wherein at least a portion of the micro-holes extend at a non-perpendicular slant angle from the outer surface to an interior of the tubular structure.

14. The stent of claim 1, wherein the plurality of cavities (26, 40) are micro-slits.

15. The stent of claim 14, wherein the micro-slits have a cross section that is smaller than a cross section of a strut.

16. The stent of claim 1, further comprising micro-slits extending from the outer surface to an interior of the tubular structure without extending through the inner surface.

17. The stent of claim 14, wherein at least a portion of the micro-slits have geometries that are configured to provide a reservoir for a coating substance applied to the tubular structure.

18. The stent of claim 16, wherein at least a portion of the micro-slits have a width of at least 17.78 µm (0.0007 inch).

19. The stent of claim 14, wherein at least a portion of the micro-slits have a width greater than 17.78 µm (0.0007 inch).

20. The stent of claim 14, wherein at least a portion of the micro-slits have a length of at least 25.4 µm (0.001 inch).

21. The stent of claim 14, wherein at least a portion of the micro-slits have a length greater than 25.4 µm (0.001 inch).

22. The stent of claim 14, wherein at least a portion of the micro-slits extend perpendicular from the outer surface to an interior of the tubular structure.

23. The stent of claim 14, wherein at least a portion of the micro-slits extend at a non-perpendicular slant angle from the outer surface to an interior of the tubular structure.

24. The stent of claim 14, wherein at least a portion of the micro-slits have a linear geometric shape.

25. The stent of claim 14, wherein at least a portion of the micro-slits have a curved geometric shape.

26. The stent of claim 1, further comprising:
• a coating substance on at least a portion of outer surface of the stent including at least a portion of the plurality of cavities (26, 40).

27. The stent of claim 26, wherein the coating substance is a restenosis inhibiting agent.

28. The stent of claim 27, wherein the restenosis inhibiting agent is selected from a drug, polymer and bio-engineered material.

29. The stent of claim 27, wherein the restenosis inhibiting agent is a combination of two agents selected from a drug, polymer and bio-engineered material.

30. The stent of claim 27, wherein each of a cavity of the plurality of cavities is configured to have a shape adapted to increase an amount of restenosis inhibiting agent coated on the stent.

31. The stent of claim 26, wherein each of a cavity of the plurality of cavities (26, 40) is configured to have a shape adapted to provide reservoir of the coated substance of the stent.

32. The stent of claim 1, comprising:
• a coating substance on at least a portion of outer surface of the stent including and extending into at least a portion of the cavities (26, 40).

33. The stent of claim 32, wherein the coating substance is on at least a portion of the inner surface of the stent.

34. The stent of claim 27, wherein the restenosis inhibiting agent is selected from a drug, polymer and bio-engineered material.

35. The stent of claim 34, wherein the restenosis inhibiting agent is a combination of two agents selected from a drug, polymer and bio-engineered material.

36. The stent of claim 33, wherein each of a cavity of the plurality of cavities (26, 40) is configured to have a shape adapted to increase an amount of restenosis inhibiting agent coated on the stent (10).

37. The stent of claim 32, wherein each of a cavity of the plurality of cavities (26, 40) is configured to have a shape adapted to provide a reservoir of the coated substance on the stent (10).

38. The stent of claim 32, wherein the tubular structure is balloon expandable.

39. The stent of claim 32, wherein the tubular structure is self-expandable.

40. The stent of claim 32, wherein at least a portion of the tubular structure is made of a shape memory alloy.

41. The stent of claim 32, wherein the plurality of cavities (26, 40) are substantially evenly positioned on the tubular structure.

42. The stent of claim 32, wherein the plurality of cavities (26, 40) increase a flexibility of the stent without substantially reducing a radial strength of the stent in a deployed state.

43. The stent of claim 32, wherein the plurality of cavities (26,40) are micro-holes.

44. The stent of claim 43, wherein the micro-holes have a cross section that is smaller than a cross section of a strut.

45. The stent of claim 32, wherein the plurality of cavities are micro-slits.

46. The stent of claim 45, wherein the micro-slits have a cross section that is smaller than a cross section of a strut.

47. The stent of claim 1, **characterized in that** the geometry of micro-slits (40) is straight linear, curvilinear, angled or squared.

48. The stent of claim 1, **characterized in that** the connector struts are curvilinear.

49. The stent of claim 1 including micro-slits of differing patterns.

50. The stent of claim 1 including micro-slits of two different signs.

51. A method of manufacturing an intravascular stent, comprising:
• forming an intravascular stent (10) having an inner surface and an outer surface, the stent having a plurality of expansion struts forming expansion columns and a plurality of connectors, the connectors connecting adjacent expansion columns; and
• forming a plurality of cavities (26, 40) on the outer surface of the intravascular stent, extending through the expansion struts and connectors from the outer surface of the stent to the inner surface of the stent, wherein the cavities formed in the connector struts are of a first size and the cavities formed in the expansion struts are of a second size different from the first.

52. The method of claim 51, wherein at least a portion of the plurality of cavities is formed on the outer surface of the intravascular stent (10) by a laser.

53. The method of claim 51, wherein the at least a portion of the plurality of cavities is formed on the outer surface of the intravascular stent (10) by electrical discharge machining process EDM.

54. The method of claim 51, wherein the at least a portion of the plurality of cavities (26, 40) is photochemically formed on the outer surface of the intravascular stent (10).

55. The method of claim 51 further comprising the step of applying a coating substance on the stent that inhibits restenosis.

## Patentansprüche

1. Ein expandierbarer Stent (10), der umfasst:
- eine röhrenförmige Struktur, die eine äußere Oberfläche, die angrenzend an eine Gefäßwand positionierbar ist, eine innere Oberfläche, die einem Hohlraum eines Körperdurchgangs zugewandt ist, mehrere Expansionsstreben (16), Verbindungsstreben (18) und Zellen (24) aufweist, wobei die Expansionsstreben (16) säulenförmige Expansionsglieder (12) bilden, wobei die röhrenförmige Struktur einen ersten Durchmesser besitzt, der die intraluminale Implantation der röhrenförmigen Struktur in den Körperdurchgang, der einen Hohlraum besitzt, ermöglicht, und einen zweiten Durchmesser in der expandierten Form des Stents besitzt, nach Anwendung einer radialen, sich nach außen erstreckenden Kraft vom Inneren der röhrenförmigen Struktur her; und
- mehrere Aushöhlungen (26, 40) die in den Expansionsstreben (16) und den Verbindungsstreben (18) des Stents ausgebildet und als Speicher einer Beschichtungssubstanz vorgesehen sind, wobei die Aushöhlungen sich von der äußeren Oberfläche des Stents durch die innere Oberfläche des Stents hindurch erstrecken, wobei in den Verbindungsstreben (18) geformte Aushöhlungen eine erste Größe besitzen, und die in den Expansionsstreben (16) geformten Aushöhlungen eine zweite Größe besitzen, die von der ersten Größe verschieden ist.

2. Der Stent nach Anspruch 1, wobei die röhrenförmige Struktur mit einem Ballon expandierbar ist.

3. Der Stent nach Anspruch 1, wobei die röhrenförmige Struktur selbst-expandierbar ist.

4. Der Stent nach Anspruch 1, wobei zumindest ein Teil der röhrenförmigen Struktur aus einer formspeichernden Legierung hergestellt ist.

5. Der Stent nach Anspruch 1, wobei die Vielzahl von Aushöhlungen (26, 40) im Wesentlichen gleichmäßig auf der röhrenförmigen Struktur positioniert ist.

6. Der Stent nach Anspruch 1, wobei die Vielzahl von Aushöhlungen (26, 40) die Flexibilität des Stents (10) erhöht, ohne die radiale Kraft des Stents in einem entfalteten Zustand wesentlich zu vermindern.

7. Der Stent nach Anspruch 1, wobei die Vielzahl der Aushöhlungen (26, 40) aus Mikrolöchern besteht, die sich von der äußeren Oberfläche aus erstrecken.

8. Der Stent nach Anspruch 7, wobei die Mikrolöcher einen Querschnitt besitzen, der kleiner ist als der Querschnitt einer Strebe.

9. Der Stent nach Anspruch 1, der außerdem Mikrolöcher aufweist, die sich von der äußeren Oberfläche bis zu einem Inneren der röhrenförmigen Struktur erstrecken, ohne durch die innere Oberfläche hindurchzuragen.

10. Der Stent nach Anspruch 7, wobei zumindest ein Teil der Mikrolöcher Geometrien besitzt, die so konfiguriert sind, dass sie einen Speicher bieten.

11. Der Stent nach Anspruch 7, wobei zumindest ein Teil der Mikrolöcher einen Durchmesser von mindesten 17,78 µm (0,0007 inch) besitzt.

12. Der Stent nach Anspruch 7, wobei sich zumindest ein Teil der Mikrolöcher senkrecht von der äußeren Oberfläche bis zu einem Inneren der röhrenförmigen Struktur erstreckt.

13. Der Stent nach Anspruch 7, wobei sich zumindest ein Teil der Mikrolöcher in einem nicht-senkrechten, schrägen Winkel von der äußeren Oberfläche bis zu einem Inneren der röhrenförmigen Struktur erstreckt.

14. Der Stent nach Anspruch 1, wobei die Mehrheit von Aushöhlungen (26, 40) Mikroschlitze sind.

15. Der Stent nach Anspruch 14, wobei die Mikroschlitze einen Querschnitt besitzen, der kleiner ist als ein Querschnitt einer Strebe.

16. Der Stent nach Anspruch 1, der außerdem Mikroschlitze aufweist, die sich von der äußeren Oberfläche zu einem Inneren der röhrenförmigen Struktur erstrecken, ohne durch die innere Oberfläche hindurchzuragen.

17. Der Stent nach Anspruch 14, wobei zumindest ein Teil der Mikroschlitze Geometrien besitzt, die so konfiguriert sind, dass sie einen Speicher für eine Beschichtungssubstanz liefern, die auf die röhrenförmige Struktur aufgetragen wird.

18. Der Stent nach Anspruch 16, wobei zumindest ein Teil der Mikroschlitze eine Breite von mindestens 17,78 µm (0,0007 inch) besitzt.

19. Der Stent nach Anspruch 14, wobei zumindest ein Teil der Mikroschlitze eine Breite besitzt, die größer als 17,78 µm (0,0007 inch) ist.

20. Der Stent nach Anspruch 14, wobei zumindest ein Teil der Mikroschlitze eine Länge von mindestens 25,4 µm (0,001 inch) besitzt.

21. Der Stent nach Anspruch 14, wobei zumindest ein Teil der Mikroschlitze eine Länge von mehr als 25,4 µm (0,001 inch) besitzt.

22. Der Stent nach Anspruch 14, wobei sich zumindest ein Teil der Mikroschlitze senkrecht von der äußeren Oberfläche zu einem Inneren der röhrenförmigen Struktur erstreckt.

23. Der Stent nach Anspruch 14, wobei sich zumindest ein Teil der Mikroschlitze in einem nicht-senkrechten, schrägen Winkel von der äußeren Oberfläche zu einem Inneren der röhrenförmigen Struktur erstreckt.

24. Der Stent nach Anspruch 14, wobei zumindest ein Teil der Mikroschlitze eine lineare geometrische Form besitzt.

25. Der Stent nach Anspruch 14, wobei zumindest ein Teil der Mikroschlitze eine gekrümmte geometrische Form besitzt.

26. Der Stent nach Anspruch 1, der außerdem umfasst:
eine Beschichtungssubstanz auf zumindest einem Teil der äußeren Oberfläche des Stents, wobei dieser Teil der äußeren Oberfläche mindestens einen Teil der Vielzahl der Aushöhlungen (26, 40) besitzt.

27. Der Stent nach Anspruch 26, wobei die Beschichtungssubstanz ein Wirkstoff ist, der die Restenose hemmt.

28. Der Stent nach Anspruch 27, wobei der die Restenose hemmende Wirkstoff aus einem Medikament, aus Polymer und aus biotechnischem Material ausgewählt wird.

29. Der Stent nach Anspruch 27, wobei der die Restenose hemmende Wirkstoff in einer Kombination aus zwei Wirkstoffen besteht, die aus einem Medikament, aus Polymer und aus biotechnischem Material ausgewählt sind.

30. Der Stent nach Anspruch 27, wobei eine jede der Aushöhlungen aus der Vielzahl der Aushöhlungen so konfiguriert ist, das sie eine Form besitzt, die dafür geeignet ist, eine Menge von Restenose hemmenden Wirkstoff zu vergrößern, die auf dem Stent aufgetragen ist.

31. Der Stent nach Anspruch 26, wobei eine jede Aushöhlung aus der Vielzahl von Aushöhlungen (26, 40) so konfiguriert ist, das sie eine Form besitzt, die dafür geeignet ist, einen Speicher der aufgetragenen Substanz des Stents bereitzustellen.

32. Der Stent nach Anspruch 1, der umfasst:
eine Beschichtungssubstanz auf zumindest einem Teil der äußeren Oberfläche des Stents, die zumindest einen Teil der Aushöhlungen (26, 40) umfasst und sich in zumindest einem Teil von ihnen erstreckt.

33. Der Stent nach Anspruch 32, wobei die Beschichtungssubstanz sich auf mindestens einem Teil der inneren Oberfläche des Stents befindet.

34. Der Stent nach Anspruch 27, wobei der die Restenose hemmende Wirkstoff aus einem Medikament, aus Polymer und aus biotechnischem Material ausgewählt ist.

35. Der Stent nach Anspruch 34, wobei der die Restenose hemmende Wirkstoff in einer Kombination aus zwei Wirkstoffen besteht, die aus einem Medikament, aus Polymer und aus biotechnischem Material ausgewählt sind.

36. Der Stent nach Anspruch 33, wobei eine jede Aushöhlung aus der Vielzahl von Aushöhlungen (26, 40) so konfiguriert ist, das sie eine Form besitzt, die dafür geeignet ist, eine Menge von Restenose hemmenden Wirkstoff zu vergrößern, die auf dem Stent (10) aufgetragen ist.

37. Der Stent nach Anspruch 32, wobei eine jede Aushöhlung aus der Vielzahl von Aushöhlungen (26, 40) so konfiguriert ist, das sie eine Form besitzt, die dafür geeignet ist, einen Speicher der aufgetragenen Substanz auf dem Stent (10) bereitzustellen.

38. Der Stent nach Anspruch 32, wobei die röhrenförmige Struktur mit einem Ballon expandierbar ist.

39. Der Stent nach Anspruch 32, wobei die röhrenförmige Struktur selbst-expandierbar ist.

40. Der Stent nach Anspruch 32, wobei zumindest ein Teil der röhrenförmigen Struktur aus einer formspeichernden Legierung hergestellt ist.

41. Der Stent nach Anspruch 32, wobei die Vielzahl von Aushöhlungen (26, 40) im Wesentlichen gleichmäßig auf der röhrenförmigen Struktur positioniert ist.

42. Der Stent nach Anspruch 32, wobei die Vielzahl von Aushöhlungen (26, 40) die Flexibilität des Stents erhöht, ohne die radiale Kraft des Stents in einem entfalteten Zustand wesentlich zu vermindern.

43. Der Stent nach Anspruch 32, wobei die Vielzahl von Aushöhlungen (26, 40) aus Mikrolöchern besteht.

44. Der Stent nach Anspruch 43, wobei die Mikrolöcher einen Querschnitt besitzen, der kleiner ist als der Querschnitt einer Strebe.

45. Der Stent nach Anspruch 32, wobei die Vielzahl der Aushöhlungen aus Mikroschlitzen besteht.

46. Der Stent nach Anspruch 45, wobei die Mikroschlitze einen Querschnitt besitzen, der kleiner ist als der Querschnitt einer Strebe.

47. Der Stent nach Anspruch 1, der **dadurch gekennzeichnet ist, dass** die Geometrie der Mikroschlitze (40) gerade linear, gekrümmt, winkelig, oder quadratisch ist.

48. Der Stent nach Anspruch 1, der **dadurch gekennzeichnet ist, dass** die Verbindungsstreben gekrümmt sind.

49. Der Stent nach Anspruch 1, der Mikroschlitze verschiedener Muster enthält.

50. Der Stent nach Anspruch 1, der Mikroschlitze zweier verschiedener Zeichen enthält.

51. Ein Verfahren zur Herstellung eines intravaskulären Stents, das umfasst:
- das Formen eines intravaskulären Stents (10), der eine innere Oberfläche und eine äußere Oberfläche besitzt, wobei der Stent eine Vielzahl von Expansionsstreben, die säulenförmige Expansionsglieder bilden, und eine Vielzahl von Verbindungsstücken besitzt, wobei die Verbindungsstücke benachbarte säulenförmige Expansionsglieder miteinander verbinden, und
- das Formen einer Vielzahl von Aushöhlungen (26, 40) auf der äußeren Oberfläche des intravaskulären Stents, die sich durch die Expansionsstreben und die Verbindungsstücke hindurch von der äußeren Oberfläche des Stents zur inneren Oberfläche des Stents erstrecken, wobei in den Verbindungsstreben geformte Aushöhlungen eine erste Größe besitzen, und die in den Expansionsstreben geformten Aushöhlungen eine zweite Größe besitzen, die von der ersten Größe verschieden ist.

52. Das Verfahren nach Anspruch 51, wobei zumindest ein Teil der Vielzahl von Aushöhlungen auf der äußeren Oberfläche des intravaskulären Stents (10) durch einen Laser gebildet wird.

53. Das Verfahren nach Anspruch 51, wobei zumindest ein Teil der Vielzahl von Aushöhlungen auf der äußeren Oberfläche des intravaskulären Stents (10) durch elektroerosive Bearbeitung (EDM) gebildet wird.

54. Das Verfahren nach Anspruch 51, wobei zumindest ein Teil der Vielzahl von Aushöhlungen (26, 40) auf der äußeren Oberfläche des intravaskulären Stents (10) photochemisch gebildet wird.

55. Das Verfahren nach Anspruch 51, das außerdem die Bearbeitungsstufe des Auftragens einer Restenose hemmenden Beschichtungssubstanz auf den Stent umfasst.

## Revendications

1. Stent expansible (10) comprenant :
une structure tubulaire comprenant une surface externe pouvant être positionnée de manière adjacente à une paroi de vaisseau, une surface interne faisant face à une lumière d'une voie de passage corporelle, une pluralité d'entretoises d'expansion (16), d'entretoises formant connecteur (18) et de cellules (24), les entretoises d'expansion (16) formant des colonnes d'expansion (12), la structure tubulaire ayant un premier diamètre qui permet la pose intraluminale de la structure tubulaire dans la voie de passage corporelle ayant une lumière, et un deuxième diamètre dans la forme expansée du stent suite à l'application depuis l'intérieur de la structure tubulaire d'une force s'étendant radialement vers l'extérieur ; et
une pluralité de cavités (26, 40) formées dans les entretoises d'expansion (16) et les entretoises formant connecteur (18) du stent servant de réservoirs d'une substance de revêtement, dans lequel les cavités s'étendent à partir de la surface externe du stent à travers la surface interne du stent, dans lequel les cavités formées dans les entretoises de connexion (18) ont une première taille et les cavités dans les entretoises d'expansion (16) ont une deuxième taille différente de la première.

2. Stent selon la revendication 1, dans lequel la structure tubulaire est expansible par ballonnet.

3. Stent selon la revendication 1, dans lequel la structure tubulaire est auto-expansible.

4. Stent selon la revendication 1, dans lequel au moins une partie de la structure tubulaire est réalisée à partir d'un alliage à mémoire de forme.

5. Stent selon la revendication 1, dans lequel la pluralité de cavités (26, 40) sont positionnées de manière sensiblement régulière sur la structure tubulaire.

6. Stent selon la revendication 1, dans lequel la pluralité de cavités augmente une flexibilité du stent (10) sans réduire sensiblement une résistance radiale du stent dans un état déployé.

7. Stent selon la revendication 1, dans lequel la pluralité de cavités (26, 40) sont des micro-trous qui s'étendent à partir de la surface externe.

8. Stent selon la revendication 7, dans lequel les micro-trous ont une section transversale qui est plus petite qu'une section transversale d'une entretoise.

9. Stent selon la revendication 1, comprenant en outre des micro-trous s'étendant à partir de la surface externe jusqu'à un intérieur de la structure tubulaire sans s'étendre à travers la surface interne.

10. Stent selon la revendication 7, dans lequel au moins une partie des micro-trous a des géométries qui sont configurées pour servir de réservoir.

11. Stent selon la revendication 7, dans lequel au moins une partie des micro-trous ont un diamètre d'au moins 17,78 µm (0,0007 pouce).

12. Stent selon la revendication 7, dans lequel au moins une partie des micro-trous s'étend perpendiculairement de la surface externe jusqu'à un intérieur de la structure tubulaire.

13. Stent selon la revendication 7, dans lequel au moins une partie des micro-trous s'étend selon un angle d'inclinaison non perpendiculaire à partir de la surface externe jusqu'à un intérieur de la structure tubulaire.

14. Stent selon la revendication 1, dans lequel la pluralité de cavités (26, 40) sont des micro-fentes.

15. Stent selon la revendication 14, dans lequel les micro-fentes ont une section transversale qui est inférieure à une section transversale d'une entretoise.

16. Stent selon la revendication 1, comprenant en outre des micro-fentes s'étendant à partir de la surface externe jusqu'à un intérieur de la structure tubulaire sans s'étendre à travers la surface interne.

17. Stent selon la revendication 14, dans lequel au moins une partie des micro-fentes a des géométries qui sont configurées pour servir de réservoir pour une substance de revêtement appliquée sur la structure tubulaire.

18. Stent selon la revendication 16, dans lequel au moins une partie des micro-fentes a une largeur d'au moins 17,78 µm (0,0007 pouce).

19. Stent selon la revendication 14, dans lequel au moins une partie des micro-fentes a une largeur supérieure à 17,78 µm (0,0007 pouce).

20. Stent selon la revendication 14, dans lequel au moins une partie des micro-fentes a une longueur d'au moins 25,4 µm (0,001 pouce).

21. Stent selon la revendication 14, dans lequel au moins une partie des micro-fentes a une longueur supérieure à 25,4 µm (0,001 pouce).

22. Stent selon la revendication 14, dans lequel au moins une partie des micro-fentes s'étend perpendiculairement de la surface externe jusqu'à un intérieur de la structure tubulaire.

23. Stent selon la revendication 14, dans lequel au moins une partie des micro-fentes s'étend selon un angle d'inclinaison non perpendiculaire de la surface externe jusqu'à un intérieur de la structure tubulaire.

24. Stent selon la revendication 14, dans lequel au moins une partie des micro-fentes a une forme géométrique linéaire.

25. Stent selon la revendication 14, dans lequel au moins une partie des micro-fentes a une forme géométrique incurvée.

26. Stent selon la revendication 1, comprenant en outre :
une substance de revêtement sur au moins une partie de surface externe du stent comprenant au moins une partie de la pluralité de cavités (26, 40).

27. Stent selon la revendication 26, dans lequel la substance de revêtement est un agent d'inhibition de resténose.

28. Stent selon la revendication 27, dans lequel l'agent d'inhibition de resténose est choisi parmi un médicament, un polymère, un matériau résultant de la bio-ingénierie.

29. Stent selon la revendication 27, dans lequel l'agent d'inhibition de resténose est une combinaison de deux agents choisis parmi un médicament, un polymère et un matériau résultant de la bio-ingénierie.

30. Stent selon la revendication 27, dans lequel chacune de la pluralité de cavités est configurée pour avoir une forme adaptée pour augmenter une quantité d'agent d'inhibition de resténose appliquée sur le stent.

31. Stent selon la revendication 26, dans lequel chacune de la pluralité de cavités (26, 40) est configurée pour avoir une forme adaptée pour servir de réservoir à la substance de revêtement du stent.

32. Stent selon la revendication 1, comprenant :
une substance de revêtement sur au moins une partie de surface externe du stent comprenant et s'étendant dans au moins une partie des cavités (26, 40).

33. Stent selon la revendication 32, dans lequel la substance de revêtement est sur au moins une partie de la surface interne du stent.

34. Stent selon la revendication 27, dans lequel l'agent d'inhibition de resténose est choisi parmi un médicament, un polymère et un matériau résultant de la bio-ingénierie.

35. Stent selon la revendication 34, dans lequel l'agent d'inhibition de resténose est une combinaison de deux agents choisis parmi un médicament, un polymère et un matériau résultant de la bio-ingénierie.

36. Stent selon la revendication 33, dans lequel chacune de la pluralité de cavités (26, 40) est configurée pour avoir une forme adaptée pour augmenter une quantité de l'agent d'inhibition de resténose appliqué sur le stent (10).

37. Stent selon la revendication 32, dans lequel chacune de la pluralité de cavités (26, 40) est configurée pour avoir une forme adaptée pour servir de réservoir à la substance appliquée sur le stent (10).

38. Stent selon la revendication 32, dans lequel la structure tubulaire est expansible par ballonnet.

39. Stent selon la revendication 32, dans lequel la structure tubulaire est auto-expansible.

40. Stent selon la revendication 32, dans lequel au moins une partie de la structure tubulaire est réalisée à partir d'un alliage à mémoire de forme.

41. Stent selon la revendication 32, dans lequel la pluralité de cavités (26, 40) sont positionnées de manière sensiblement régulière sur la structure tubulaire.

42. Stent selon la revendication 32, dans lequel la pluralité de cavités (26, 40) augmentent une flexibilité du stent sans réduire sensiblement une résistance radiale du stent dans un état déployé.

43. Stent selon la revendication 32, dans lequel la pluralité de cavités (26, 40) sont des micro-trous.

44. Stent selon la revendication 43, dans lequel les micro-trous ont une section transversale qui est inférieure à une section transversale d'une entretoise.

45. Stent selon la revendication 32, dans lequel la pluralité de cavités sont des micro-fentes.

46. Stent selon la revendication 45, dans lequel les micro-fentes ont une section transversale qui est inférieure à une section transversale d'une entretoise.

47. Stent selon la revendication 1, **caractérisé en ce que** la géométrie des micro-fentes (40) est linéaire droite, curviligne, coudée ou carrée.

48. Stent selon la revendication 1, **caractérisé en ce que** les entretoises de connexion sont curvilignes.

49. Stent selon la revendication 1, comprenant des micro-fentes de différents modèles.

50. Stent selon la revendication 1, comprenant des micro-fentes de deux signes différents.

51. Procédé pour fabriquer un stent intravasculaire, comprenant les étapes consistant à :
former un stent intravasculaire (10) ayant une surface interne et une surface externe, le stent ayant une pluralité d'entretoises d'expansion formant des colonnes d'expansion et une pluralité de connecteurs, les connecteurs raccordant les colonnes d'expansion adjacentes ; et
former une pluralité de cavités (26, 40) sur la surface externe du stent intravasculaire, s'étendant à travers les entretoises d'expansion et les connecteurs à partir de la surface externe du stent jusqu'à la surface interne du stent, dans lequel les cavités formées dans les entretoises formant connecteurs ont une première taille et les cavités formées dans les entretoises d'expansion ont une deuxième taille différente de la première.

52. Procédé selon la revendication 51, dans lequel au moins une partie de la pluralité de cavités est formée sur la surface externe du stent intravasculaire (10) par un laser.

53. Procédé selon la revendication 51, dans lequel au moins une partie de la pluralité de cavités est formée sur la surface externe du stent intravasculaire (10) par un procédé d'usinage par décharge électrique EDM.

54. Procédé selon la revendication 51, dans lequel la au moins une partie de la pluralité de cavités (26, 40) est formée de manière photochimique sur la surface externe du stent intravasculaire (10).

55. Procédé selon la revendication 51 comprenant en outre l'étape consistant à appliquer une substance de revêtement sur le stent qui empêche la resténose.
